# EUROPÄISCHE PATENTANMELDUNG

(11) **EP 1 826 497 A2**
(43) Veröffentlichungstag der Anmeldung: **29.08.2007**
(21) Anmeldenummer: 07102453.3
(22) Anmeldetag: 15.02.2007
(51) Int. Cl.: F24F 3/16

(54) **Verfahren zur Aufbereitung eines von einer raumlufttechnischen Anlage in den Raum eines Gebäudes eingeleiteten Luftstromes**

(30) Priorität: 22.02.2006 DE 102006008265
(71) Anmelder: Laskowski, Rainer, Dipl.-Ing., 32130 Enger (DE); Kolb, Thomas, 90571 Schwaig (DE); Lehmann, Jörg, Dipl.-Ing., 01723 Kesselsdorf (DE)
(72) Erfinder: Laskowski, Rainer, Dipl.-Ing., 32130 Enger (DE); Kolb, Thomas, 90571 Schwaig (DE); Lehmann, Jörg, Dipl.-Ing., 01723 Kesselsdorf (DE)
(74) Vertreter: Zech, Stefan Markus

(57) **Zusammenfassung**

Die Erfindung betrifft ein Verfahren zur Aufbereitung eines von einer raumlufttechnischen Anlage (1) in den Raum (2) eines Gebäudes eingeleiteten Luftstroms, wobei der Luftstrom vor seiner Ausleitung in den Raum (2) des Gebäudes einer Sauerstoffaktivierung unterzogen wird. Um in Raum sparender Weise und möglichst autark die Sauerstoffaktivierung vornehmen zu können, sieht die Erfindung vor, dass der Luftstrom von der raumlufttechnischen Anlage (1) zunächst in eine als kompakte Einheit (3) ausgebildete Sauerstoffaktivierungs-Vorrichtung eingeleitet wird, in der eine Ionenerzeugung und/oder eine Ozonerzeugung erfolgt, und dass der so aufbereitete Luftstrom dann von der kompakten Einheit (3) in den Raum (2) des Gebäudes ausgeleitet wird. Des weiteren betrifft die Erfindung eine Vorrichtung zur Aufbereitung des von der raumlufttechnischen Anlage in den Raum eingeleiteten Luftstroms.

## Beschreibung

Die Erfindung betrifft ein Verfahren zur Aufbereitung eines von einer raumlufttechnischen Anlage in den Raum eines Gebäudes eingeleiteten Luftstroms, wobei der Luftstrom vor seiner Ausleitung in den Raum des Gebäudes einer Sauerstoffaktivierung unterzogen wird. Des weiteren betrifft die Erfindung eine Vorrichtung zur Aufbereitung des von der raumlufttechnischen Anlage in den Raum eingeleiteten Luftstroms.

Verfahren zur Aufbereitung eines in einen Raum einzuleitenden Luftstroms sind im Stand der Technik bekannt, wozu beispielsweise auf die DE 10 2004 003 269 A1 und auf die DE 20 2004 012 352 U1 hingewiesen wird.

Um die Luftqualität zu verbessern, wird dabei eine Sauerstoffaktivierung derart durchgeführt, dass die zuzuleitende Raumluft mit Ionen und/oder mit Ozon angereichert wird. Auf diese Weise wird die Qualität der Atemluft mit Blick auf das Wohlbefinden und die Gesundheit verbessert. Ferner kann dadurch einer frühzeitigen Erschöpfung des im Raum tätigen Menschen und anderen Mängeln (z. B. Allergien) entgegengewirkt werden.

Nachteilig ist es, dass vorbekannte Anlagen sehr aufwändig ausgestaltet sind und eine Vielzahl von Komponenten aufweisen, was nicht nur zu einem hohen Montageaufwand führt, sondern auch einen hohen Raumbedarf zur Folge hat. Weiterhin setzen vorbekannte Verfahren und zugehörige Vorrichtungen das Vorhandensein einer entsprechenden Infrastruktur voraus, was insbesondere mit Blick auf die Versorgung mit elektrischer Energie gilt.

Der Erfindung liegt daher die Aufgabe zugrunde, ein Verfahren zur Aufbereitung eines von einer raumlufttechnischen Anlage in den Raum eines Gebäudes eingeleiteten Luftstroms der eingangs genannten Art sowie eine zugehörige Vorrichtung zu schaffen, das bzw. die es ermöglicht, die genannten Nachteile zu beheben bzw. zu vermindern. Namentlich soll die Luftaufbereitung in Raum sparender Weise und montagetechnisch einfach möglich werden. Ein besonderer Aspekt ist weiterhin, dass der Einsatz des Verfahrens und der Vorrichtung auch möglich sein soll, wenn schlechte Verhältnisse bezüglich der Infrastruktur der Energieversorgung vorliegen.

Die **Lösung** dieser Aufgabe durch die Erfindung ist verfahrensgemäß dadurch gekennzeichnet, dass der Luftstrom von der raumlufttechnischen Anlage zunächst in eine als kompakte Einheit ausgebildete Sauerstoffaktivierungs-Vorrichtung eingeleitet wird, in der eine Ionenerzeugung und/oder eine Ozonerzeugung erfolgt, und dass der so aufbereitete Luftstrom dann von der kompakten Einheit in den Raum des Gebäudes ausgeleitet wird.

Dabei ist bevorzugt vorgesehen, dass die Sauerstoffaktivierung in der kompakten Einheit in einer autarken Weise und weitgehend, vorzugsweise vollständig, ohne externe Energieversorgung durchgeführt wird. Dies ermöglicht den Einsatz des Verfahrens an einem Ort, der nicht über die infrastrukturellen Voraussetzungen für klassische Laufaufbereitungsanlagen verfügt.

Für die Sauerstoffaktivierung sind mehrere Möglichkeiten denkbar: Nach einer ersten Ausgestaltung des Verfahrens findet in der kompakten Einheit nur eine Ionenerzeugung statt. Alternativ hierzu kann dort nur eine Ozonerzeugung stattfinden. Möglich ist es aber auch, dass in der kompakten Einheit sowohl eine Ionenerzeugung als auch eine Ozonerzeugung stattfindet.

Im Falle der Sauerstoffanreicherung mittels Ionenerzeugung ist bevorzugt vorgesehen, dass die Ionenerzeugung mit einer Rate von mindestens 2 Millionen Ionen / sec, vorzugsweise von 4 Millionen Ionen /sec, erfolgt. Wird auf die Ozonerzeugung abgestellt, hat es sich als vorteilhaft erwiesen, wenn diese mit einer Rate von mindestens 15 mg/Stunde, vorzugsweise von 25 mg/Stunde, erfolgt.

Die erfindungsgemäße Vorrichtung zur Aufbereitung eines von einer raumlufttechnischen Anlage in den Raum eines Gebäudes eingeleiteten Luftstroms, wobei die Vorrichtung Mittel zur Sauerstoffaktivierung aufweist, zeichnet sich dadurch aus, dass die Vorrichtung als kompakte Einheit ausgebildet ist, in der die Mittel zur Sauerstoffaktivierung angeordnet sind, wobei die Mittel zur Ionenerzeugung und/oder zur Ozonerzeugung ausgebildet sind.

Mit besonderem Vorteil weist die Vorrichtung ein Speichermittel für elektrische Energie auf, wobei hierbei insbesondere an einen Akkumulator, speziell an einen 12-Volt-Akkumulator, gedacht ist.

Die kompakte Einheit ist bevorzugt in einem im Wesentlichen quaderförmigen Gehäuse angeordnet, das an der Decke eines Raums befestigt werden kann.

Die Mittel zur Ionenerzeugung und/oder zur Ozonerzeugung können einen Ionenerzeuger umfassen, der mit einem Hochspannungserzeuger in Verbindung steht. Entsprechend können diese Mittel einen Ozonerzeuger umfassen, der ebenfalls mit einem Hochspannungserzeuger in Verbindung steht.

Die Vorrichtung kann Signalmittel aufweisen, die den Ladezustand der Speichermittel für elektrische Energie, also des Akkumulators, angeben. Dabei kann ein Signalmittel als optische Anzeige und ein Signalmittel als akustische Anzeige ausgebildet sein. Vorgesehen werden können auch Signalmittel zur Angabe des Betriebszustands und/oder des Betriebsmodus der Vorrichtung.

In einer bevorzugten Ausgestaltung der vorliegenden Erfindung umfasst die Vorrichtung eine Luftstromüberwachungs-Einrichtung. Die Luftstromüberwachs-Einrichtung erfasst, ob eine bzw. eine ausreichende Luftströmung im Luftleitkanal vorhanden ist bzw. misst die Strömungsgeschwindigkeit der Luft im Luftleitkanal. Hierzu kann ein Luftgeschwindigkeitssensor vorgesehen sein. Die Luftstromüberwachungseinrichtung kann wahlweise innerhalb der kompakten Einheit der Sauerstoffaktivierungs-Vorrichtung, zustrom- oder abstromseitig der Sauerstoffaktivierungs-Vorrichtung vorgesehen sein. In einer ersten Ausgestaltung ist vorgesehen, dass die Sauerstoffaktivierung erst ab einer vorbestimmten Luftströmung einsetzt. Dabei steht die Luftstromüberwachungseinrichtung mit einer Steuerung, vorzugsweise in Gestalt einer Elektronik, in Wirkverbindung, um die Sauerstoffaktivierung zu- bzw. abzuschalten. In einer alternativen Ausgestaltung wird die Menge der erzeugten Ionen und/oder die Menge des erzeugten Ozons in Abhängigkeit von der Luftgeschwindigkeit kontinuierlich eingestellt.

Vom Schutz der vorliegenden Anmeldung umfasst ist auch eine Anordnung, bestehend aus einer raumlufttechnischen Anlage und der genannten Vorrichtung zur Sauerstoffaktivierung.

Dabei kann im einfachsten Falle die raumlufttechnische Anlage ein Luftleitkanal sein, der eine fluidische Verbindung zwischen einer Frischluftquelle und der Sauerstoffaktivierungs-Vorrichtung herstellt.

In technisch aufwändigeren Fällen ist die raumlufttechnische Anlage eine Lüftungsanlage oder eine Klimaanlage. Die Klimaanlage kann - in bekannter Weise -Mittel zum Erwärmen von Luft, Mittel zum Kühlen von Luft und/oder Mittel zum Befeuchten von Luft aufweisen.

Bevorzugt ist vorgesehen, dass die Verbindung zwischen der raumlufttechnischen Anlage und der Sauerstoffaktivierungs-Vorrichtung durch einen Luftleitkanal hergestellt wird.

Mit der vorgeschlagenen Lösung wird es möglich, in effizienter Weise eine Sauerstoffaktivierung von zuzuleitender Raumluft vorzunehmen, ohne dass ein hoher Raumbedarf besteht und ggf. auch, ohne dass eine separate Energieversorgung zur Verfügung steht.

Die vorgeschlagene kompakte Sauerstoffaktivierungseinheit kann an den Zuluftauslass eines Zuluftkanals einer bestehenden raumlufttechnischen Anlage angefügt werden, so dass eine universelle Anwendungsmöglichkeit besteht.

Vorteilhaft ist auch, dass der Montageaufwand der vorgeschlagenen Sauerstoffaktivierungs-Vorrichtung gering ist und die Montage damit kostengünstig vorgenommen werden kann.

In der Zeichnung sind Ausführungsbeispiele der Erfindung dargestellt. Es zeigen:
- Fig. 1: die geschnittene Seitenansicht durch einen Raum, der mit Luft zu versorgen ist,
- Fig. 2: die schematische Darstellung einer Sauerstoffanreicherungs-Vorrichtung gemäß einer ersten Ausgestaltung der Erfindung und
- Fig. 3: die schematische Darstellung einer alternativen Sauerstoffanreicherungs-Vorrichtung gemäß einer zweiten Ausgestaltung der Erfindung.

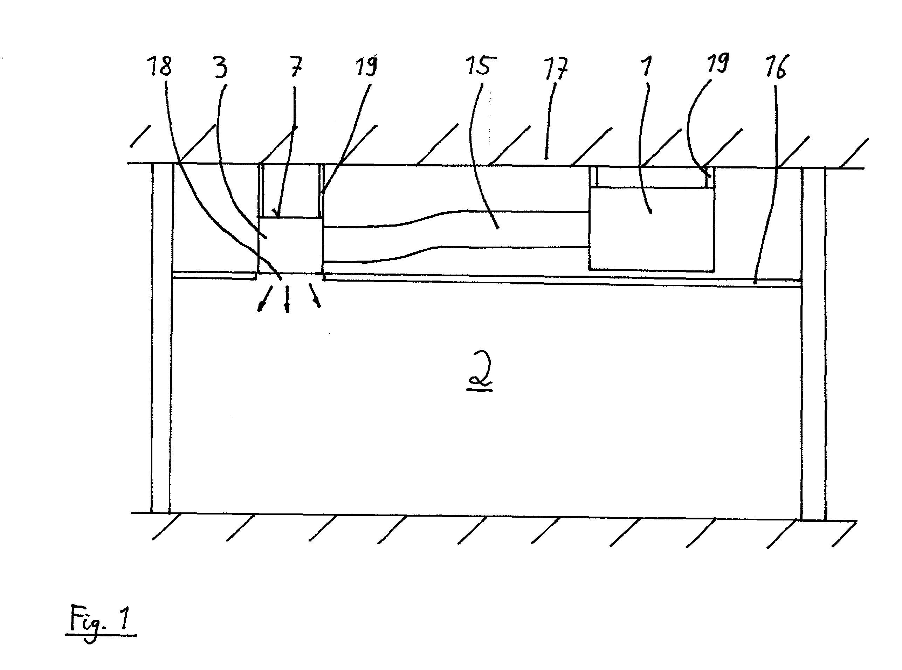

In Fig. 1 ist ein mit Luft zu versorgender Raum 2 skizziert, wobei die Luft über eine raumlufttechnische Anlage 1 zugeführt wird. Bei dieser kann es sich um eine Klima- oder Lüftungsanlage handeln, die als solche bekannt und nicht näher dargestellt ist. Der Raum 2 ist mit einer abgehängten Decke 16 ausgestattet, die unterhalb der Decke 17 angeordnet ist. Die Luft wird aus der abgehängten Decke 16 aus einem Luftauslass 18 abgegeben, wobei durch Pfeile der Luftstrom angedeutet ist.

Die in den Raum einzuleitende (Frisch-)Luft gelangt von der raumlufttechnischen Anlage 1 über einen Luftleitkanal 15 zu einer Sauerstoffaktivierungs-Vorrichtung 3, die an den Luftauslass 18 angrenzt. Sowohl ein Teil der raumlufttechnischen Anlage 1 als auch die Sauerstoffaktivierungs-Vorrichtung 3 sind mit Befestigungsmitteln 19 an der Decke 17 befestigt.

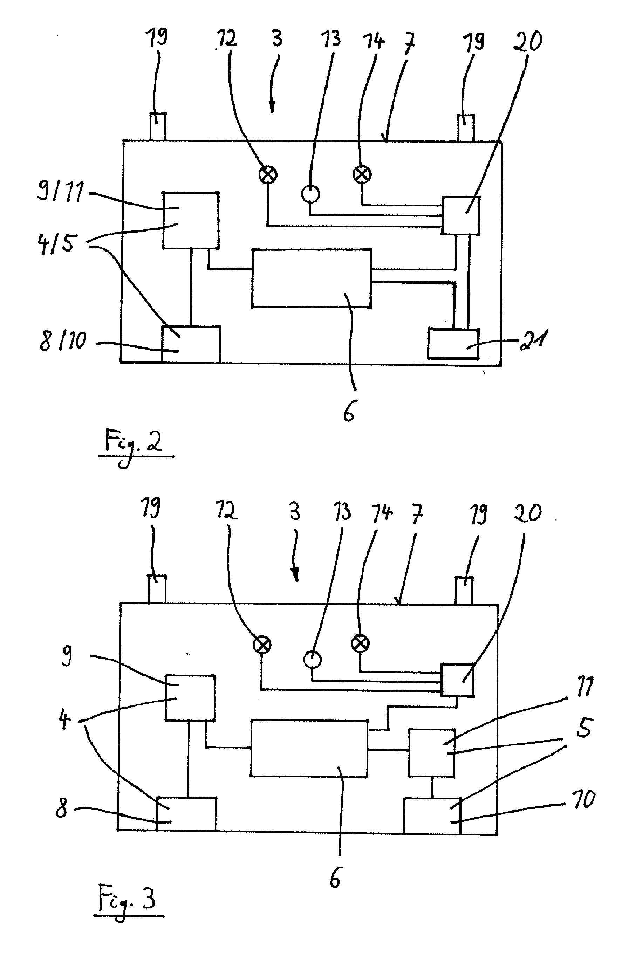

Der Aufbau der Sauerstoffaktivierungs-Vorrichtung 3 geht aus den Figuren 2 und 3 hervor, wobei hier zwei unterschiedliche Ausführungsbeispiele schematisch dargestellt sind.

Die Sauerstoffaktivierungs-Vorrichtung 3 gemäß Fig. 2 besteht - wie die gemäß Fig. 3 auch - aus einem Gehäuse 7, das im Wesentlichen quaderförmig ausgestaltet ist. Das Gehäuse 7 verleiht der Sauerstoffaktivierungs-Vorrichtung 3 eine kompakte Bauform, d. h. die Vorrichtung 3 ist als kompakte Einheit ausgebildet. Sie nimmt Luft aus der raumlufttechnischen Anlage 1 auf und gibt sie nach der Sauerstoffanreicherung an den Raum 2 ab.

Zur Sauerstoffanreicherung hat die Vorrichtung 3 gemäß Fig. 2 Mittel 4 bzw. 5. Diese Mittel können entweder zur Ionenerzeugung (Mittel 4) oder zur Ozonerzeugung (Mittel 5) ausgebildet sein.

Im einen Falle ist ein Ionenerzeuger 8 vorgesehen, der mit einem Hochspannungserzeuger 9 in Verbindung steht und von diesem die Energie für die Ionenerzeugung erhält. Im anderen Falle ist ein Ozonerzeuger 10 vorgesehen, der mit einem Hochspannungserzeuger 11 in Verbindung steht.

Der Hochspannungserzeuger 9, 11 bezieht die benötigte Energie von einem Speichermittel 6 für elektrische Energie, d. h. vorliegend von einer wieder aufladbaren Batterie (Akkumulator). Dabei handelt es sich um einen 12 Volt - Akkumulator, der auf beliebiger Basis arbeiten kann (Trockenbatterie oder Flüssigkeits-(Säure-)Batterie).

Der Ladestatus des Akkumulators 6 kann über Signalmittel 12 und 13 angezeigt werden. Dabei ist das Signalmittel 12 als Lampe oder Leuchtdiode für die optische Anzeige ausgeführt. Die zusätzlichen Signalmittel 13 sind als akustische Signalmittel ausgebildet. Ferner können weitere Signalmittel 14 - beispielsweise in Form einer oder mehrerer Lampen oder Leuchtdioden - vorgesehen werden, die den Betriebszustand bzw. den Betriebsmodus der Vorrichtung 3 angeben. Die Ansteuerung der Signalmittel 12, 13, 14 erfolgt von einer Elektronik 20, die mit dem Akkumulator 6 in Verbindung steht.

Weiter kann eine Luftstromüberwachungseinrichtung 21 vorgesehen sein, die mit einem Sensor zur direkten oder indirekten Ermittlung der Luftströmung in Wirkverbindung steht. Dieser Sensor kann als Druck- bzw. Geschwindigkeitssensor ausgebildet sein. Die Luftstromüberwachungs-Einrichtung 21 bewirkt aufgrund der Signale des Sensors eine Steuerung des Ionenerzeugers 8 bzw. Ozonerzeugers 10. Dabei kann die Steuerung ein simples Zu- bzw. Abschalten oder auch eine Einstellung, insbesondere kontinuierliche Einstellung der Ionenerzeugungs- bzw. Ozonerzeugungsrate umfassen.

Die Signalmittel 12, 13, 14 können es beispielsweise anzeigen, wenn der Akkumulator 6 nur noch einen geringen Ladestatus oder gar keine Ladung mehr aufweist. Die Signalmittel 14 können insbesondere den Betriebszustand der Vorrichtung 3 durch farblich unterschiedliche Leuchtdioden anzeigen.

Vorgesehen kann auch werden, dass sowohl eine Ionenerzeugung als auch eine Ozonerzeugung erfolgt. Eine solche Lösung ist in Fig. 3 dargestellt. Wie zu erkennen ist, versorgt hier der Akkumulator 6 zwei Hochspannungserzeuger 9 und 11, die dann einmal den Ionenerzeuger 8 und einmal den Ozonerzeuger 10 mit Spannung beaufschlagen. Wie bei der Lösung gemäß Fig. 2 gilt auch hier, dass die Ionenerzeugung und die Ozonerzeugung in den jeweiligen Mitteln 8, 10 regelbar sind, so dass die Qualität der Raumluft hinsichtlich ihrer Anreicherung bzw. Aktivierung mit Ionen bzw. Ozon an individuell gewünschte Bedürfnisse anpassbar ist.

Zur Sauerstoffaktivierung sind - wie erläutert - insbesondere die Anreicherung mit Ionen und mit Ozon vorgesehen, was als solches bekannt ist. Es wird bezüglich Details ausdrücklich insbesondere auf die DE 10 2004 003 269 A1 und auf die DE 20 2004 012 352 U1 sowie auf die in diesen Dokumenten genannten Druckschriften Bezug genommen.

Das Gehäuse 7 ist als EMV-geprüftes Modulgehäuse ausgebildet.

### Bezugszeichenliste

- 1: raumlufttechnische Anlage
- 2: Raum
- 3: Sauerstoffaktivierungs-Vorrichtung
- 4, 5: Mittel zur Sauerstoffaktivierung
- 4: Mittel zur Ionenerzeugung
- 5: Mittel zur Ozonerzeugung
- 6: Speichermittel für elektrische Energie (Akkumulator)
- 7: Gehäuse
- 8: Ionenerzeuger
- 9: Hochspannungserzeuger
- 10: Ozonerzeuger
- 11: Hochspannungserzeuger
- 12: Signalmittel (optische Anzeige)
- 13: Signalmittel (akustische Anzeige)
- 14: Signalmittel
- 15: Luftleitkanal
- 16: abgehängte Decke
- 17: Decke
- 18: Luftauslass
- 19: Befestigungsmittel
- 20: Elektronik
- 21: Luftstromüberwachungs-Einrichtung

## Patentansprüche

1. Verfahren zur Aufbereitung eines von einer raumlufttechnischen Anlage (1) in den Raum (2) eines Gebäudes eingeleiteten Luftstroms, wobei der Luftstrom vor seiner Ausleitung in den Raum (2) des Gebäudes einer Sauerstoffaktivierung unterzogen wird,
**dadurch gekennzeichnet,**
**dass** der Luftstrom von der raumlufttechnischen Anlage (1) zunächst in eine als kompakte Einheit (3) ausgebildete Sauerstoffaktivierungs-Vorrichtung eingeleitet wird, in der eine Ionenerzeugung und/oder eine Ozonerzeugung erfolgt, und dass der so aufbereitete Luftstrom dann von der kompakten Einheit (3) in den Raum (2) des Gebäudes ausgeleitet wird.

2. Verfahren nach Anspruch 1,
**dadurch gekennzeichnet,**
**dass** die Sauerstoffaktivierung in der kompakten Einheit (3) in autarker Weise und weitgehend, vorzugsweise vollständig, ohne externe Energieversorgung durchgeführt wird.

3. Verfahren nach Anspruch 1 oder 2,
**dadurch gekennzeichnet,**
**dass** in der kompakten Einheit (3) nur eine Ionenerzeugung stattfindet.

4. Verfahren nach Anspruch 1 oder 2,
**dadurch gekennzeichnet,**
**dass** in der kompakten Einheit (3) nur eine Ozonerzeugung stattfindet.

5. Verfahren nach Anspruch 1 oder 2,
**dadurch gekennzeichnet,**
**dass** in der kompakten Einheit (3) sowohl eine Ionenerzeugung als auch eine Ozonerzeugung stattfindet.

6. Verfahren nach einem der Ansprüche 1 bis 3 oder 5,
**dadurch gekennzeichnet,**
**dass** die Ionenerzeugung mit einer Rate von mindestens 2 Millionen Ionen / sec, vorzugsweise von 4 Millionen Ionen /sec, erfolgt.

7. Verfahren nach einem der Ansprüche 1, 2, 4 oder 5,
**dadurch gekennzeichnet,**
**dass** die Ozonerzeugung mit einer Rate von mindestens 15 mg/Stunde, vorzugsweise von 25 mg/Stunde, erfolgt.

8. Vorrichtung (3) zur Aufbereitung eines von einer raumlufttechnischen Anlage (1) in den Raum (2) eines Gebäudes eingeleiteten Luftstroms, wobei die Vorrichtung (3) Mittel (4, 5) zur Sauerstoffaktivierung aufweist, insbesondere zur Durchführung des Verfahrens nach einem der Ansprüche 1 bis 7,
**dadurch gekennzeichnet,**
**dass** die Vorrichtung (3) als kompakte Einheit (1) ausgebildet ist, in der die Mittel (4, 5) zur Sauerstoffaktivierung angeordnet sind, wobei die Mittel (4, 5) zur Ionenerzeugung und/oder zur Ozonerzeugung ausgebildet sind.

9. Vorrichtung nach Anspruch 8,
**dadurch gekennzeichnet,**
**dass** sie ein Speichermittel (6) für elektrische Energie aufweist.

10. Vorrichtung nach Anspruch 9,
**dadurch gekennzeichnet,**
**dass** das Speichermittel (6) für elektrische Energie als Akkumulator, insbesondere als 12-Volt-Akkumulator, ausgebildet ist.

11. Vorrichtung nach einem der Ansprüche 8 bis 10,
**dadurch gekennzeichnet,**
**dass** die kompakte Einheit (1) in einem im Wesentlichen quaderförmigen Gehäuse (7) angeordnet ist.

12. Vorrichtung nach einem der Ansprüche 8 bis 11,
**dadurch gekennzeichnet,**
**dass** die Mittel (4, 5) zur Ionenerzeugung und/oder zur Ozonerzeugung einen Ionenerzeuger (8) umfassen, der mit einem Hochspannungserzeuger (9) in Verbindung steht.

13. Vorrichtung nach einem der Ansprüche 8 bis 11,
**dadurch gekennzeichnet,**
**dass** die Mittel (4, 5) zur Ionenerzeugung und/oder zur Ozonerzeugung einen Ozonerzeuger (10) umfassen, der mit einem Hochspannungserzeuger (11) in Verbindung steht.

14. Vorrichtung nach einem der Ansprüche 9 bis 13,
**dadurch gekennzeichnet,**
**dass** die Vorrichtung Signalmittel (12, 13) aufweist, die den Ladezustand der Speichermittel (6) für elektrische Energie angeben.

15. Vorrichtung nach Anspruch 14,
**dadurch gekennzeichnet,**
**dass** mindestens ein Signalmittel (12) als optische Anzeige ausgebildet ist.

16. Vorrichtung nach Anspruch 14,
**dadurch gekennzeichnet,**
**dass** mindestens ein Signalmittel (13) als akustische Anzeige ausgebildet ist.

17. Vorrichtung nach einem der Ansprüche 8 bis 16,
**dadurch gekennzeichnet,**
**dass** die Vorrichtung Signalmittel (14) zur Angabe des Betriebszustands und/oder des Betriebsmodus der Vorrichtung (3) aufweisen.

18. Anordnung, bestehend aus einer raumlufttechnischen Anlage (1) und einer Vorrichtung (3) nach einem der Ansprüche 8 bis 17.

19. Anordnung nach Anspruch 18,
**dadurch gekennzeichnet,**
**dass** die raumlufttechnische Anlage (1) ein Luftleitkanal ist, der eine fluidische Verbindung zwischen einer Frischluftquelle und der Vorrichtung (3) herstellt.

20. Anordnung nach Anspruch 18,
**dadurch gekennzeichnet,**
**dass** die raumlufttechnische Anlage (1) eine Lüftungsanlage ist.

21. Anordnung nach Anspruch 18,
**dadurch gekennzeichnet,**
**dass** die raumlufttechnische Anlage (1) eine Klimaanlage ist.

22. Anordnung nach Anspruch 21,
**dadurch gekennzeichnet,**
**dass** die Klimaanlage Mittel zum Erwärmen von Luft aufweist.

23. Anordnung nach Anspruch 21,
**dadurch gekennzeichnet,**
**dass** die Klimaanlage Mittel zum Kühlen von Luft aufweist.

24. Anordnung nach Anspruch 21,
**dadurch gekennzeichnet,**
**dass** die Klimaanlage Mittel zum Befeuchten von Luft aufweist.

25. Anordnung nach einem der Ansprüche 20 bis 24,
**dadurch gekennzeichnet,**
**dass** die Verbindung zwischen der raumlufttechnischen Anlage (1) und der Vorrichtung (3) durch einen Luftleitkanal (15) hergestellt wird.
